Europäisches Patentamt

European Patent Office

Office européen des brevets

(11) Publication number: **0 172 036 B1**

# EUROPEAN PATENT SPECIFICATION

(45) Date of publication of patent specification: **24.04.91**  (51) Int. Cl.⁵: **A61F 13/15**

(21) Application number: **85305845.1**

(22) Date of filing: **16.08.85**

(54) **Infant diaper with improved fit.**

(30) Priority: **17.08.84 US 641666**

(43) Date of publication of application:
**19.02.86 Bulletin  86/08**

(45) Publication of the grant of the patent:
**24.04.91 Bulletin  91/17**

(84) Designated Contracting States:
**AT BE CH DE FR GB IT LI LU NL SE**

(56) References cited:
**EP-A- 0 112 655
US-A- 4 407 284
US-A- 4 430 086**

(73) Proprietor: **WEYERHAEUSER COMPANY**

**Tacoma, WA 98477(US)**

(72) Inventor: **Huffman, Gloria**
**Box 194**
**Skillman New Jersey(US)**
Inventor: **Pieniak, Heinz A.**
**12, Nathan Drive**
**North Brunswick, N.J. 08902(US)**

(74) Representative: **Jones, Alan John et al**
**CARPMAELS & RANSFORD 43 Bloomsbury
Square**
**London, WC1A 2RA(GB)**

## Description

The present invention relates to a new and improved disposable infant diaper. The diaper has improved fit about the waist and the legs of the infant.

Disposable absorbent products have been known for some time including such products as disposable diapers and incontinence pads. These products incorporate an absorbent batt which is used to absorb and hold or contain body fluids. Initially in many of these products, especially diapers and sanitary napkins, the absorbent batt comprised what is termed wadding or plies of tissue. The wadding was disposed between a liquid-impermeable backing and a liquid-permeable facing and the plies of tissue were used to absorb and hopefully contain the liquid within the product. A diaper which utilizes such an absorbent batt is disclosed in U.S. Reissue Patent No. 26,151.

The wadding type of product was replaced for the most part by an improved absorbent batt which comprises what is termed "fluffed wood pulp fibers". This absorbent batt comprises a layer of individualized wood pulp fibers with the layer having substantial thickness. A diaper which incorporates such a fluffed wood pulp absorbent batt is described in U.S. Patent No. 2,788,003. This diaper had improved absorbent capacity and somewhat better containment than a diaper using a wadding layer. Also, the fluffed wood pulp layer is quite soft, flexible, and conformable, and hence produces an improved diaper over diapers using wadding as the absorbent layer. Though the fluffed wood pulp absorbent batt has improved capacity, diaper designs, whether they be for the infant or the adult, have followed the standard pattern of having large areas of liquid-impermeable film encompassing the torso.

When the diaper is dry, the wood pulp fluff permits the diaper to breathe to some extent. However, once the diaper is wet and the wood pulp fluff has compacted, the breathability of the diaper reaches a very low point.

Other efforts have been made to conform the diaper about the legs of the wearer to prevent leakage. Though these diapers provide no better absorbent batt than flat diapers of the prior art, they have indicated improved containment of liquid. Such diapers are disclosed and described in U.S. Patents Nos. 3,860,003; 4,050,462; and 4,324,245. The elasticized products fit more tightly, permitting less air circulation. Frequently, this can become irritating to the skin and the tighter the elastic or the more closely fitting the diaper, the greater the irritation. This is especially true adjacent the area where the elastic leg portion of the product contacts the wearer.

EP-A-0 112 655 discloses a disposable infant diaper comprising:

(a) an absorbent panel;

(b) a first breathable fabric on which the absorbent panel is located, the first breathable fabric extending beyond the longitudinal edges of the absorbent panel to form margins;

(c) a second breathable fabric covering said absorbent panel and extending beyond said panel at least sufficiently to be adhered to said first breathable fabric in the margins;

(d) elastic gathering means secured between said first and second breathable fabrics in said margins along the longitudinal length of said absorbent panel in at least the central portion thereof; and

(e) closure means attached on the inside of the diaper at one end and on either side of said diaper.

The present invention provides a new and improved disposable infant diaper product whose constituency and design is specifically for an infant. This new diaper product absorbs as much urine with less leakage as present commercial diapers, and the wearer experiences less skin irritation than with previously known products.

The disposable infant diaper of the present invention is characterised in that:

(f) the diaper has a modified "T" configuration;

(g) the absorbent panel is substantially rectangular;

(h) there is a liquid barrier between said absorbent panel and said first breathable fabric, said barrier being substantially the same shape as said panel but extending slightly beyond each edge of said panel;

(i) the liquid barrier and the absorbent panel together form an absorbent core, which core forms the stem of the "T" and extends substantially the entire length of the "T";

(j) the first breathable fabric forms the top of the "T";

(k) the closure means are attached at respective ends of the top of the "T";

(l) extended portions of at least one of said breathable fabrics are provided at the base of the "T" along a small portion of the stem of the "T" on each side in substantially equal portions; and

(m) said breathable fabrics are substantially non-extendible in a cross-wise direction.

Up to about 10 percent yielding can be tolerated. By non-yielding is meant that if the fabric is extended it does not remain extended.

The combination of the modified "T" configuration and the non-yielding fabrics provides a product which fits the infant's torso securely and yet provides a high degree of breathability. At least about 35 percent and generally more than 40 percent of the diaper product is breathable. Included in the breathable area are the elastics. This modified "T" configuration accommodates a thin absorbent core or a thick absorbent core.

In the accompanying drawings:
Figure 1 is a plan view illustrating one embodiment of the present invention; and
Figure 2 is a plan view of the embodiment of Figure 1 with special delineations showing the relationship of parts of the diaper configuration.

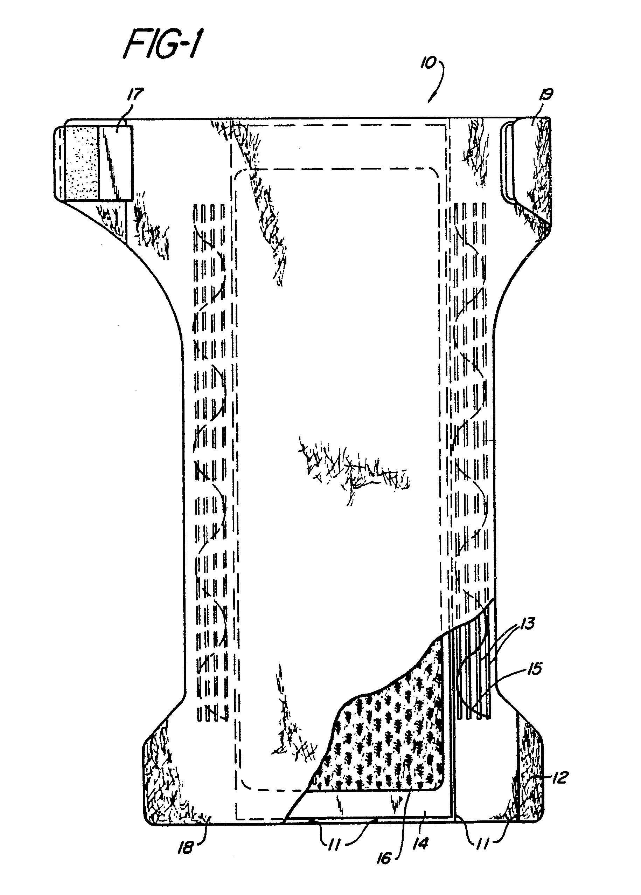

Referring now to the drawings, Figure 1 represents a top view of the disposable diaper product of the present invention. The diaper 10 has a spun bonded polypropylene breathable fabric backing 12 which is substantially in the shape of a "T" but modified as discussed hereinafter. A liquid barrier 14, such as a polyethylene film, of substantially rectangular shape, is placed on the backing 12. An absorbent panel 16 which is slightly smaller than the liquid barrier 14 is placed centrally located on the liquid barrier. Another sheet of polypropylene fabric 18 is placed on top of the product to create the facing which comes in contact with the infant's skin. In the margin, elastic strands 13 are placed alongside the liquid barrier and the absorbent panel. In this embodiment, the elastic means consists of multiple strands of elastic 13 which are adhered by the glue line 15. The diaper is secured about the body of the infant by the closure means 17 and 19. The closure 17 is shown in its open position ready to apply and closure means 19 is shown in its closed storage position.

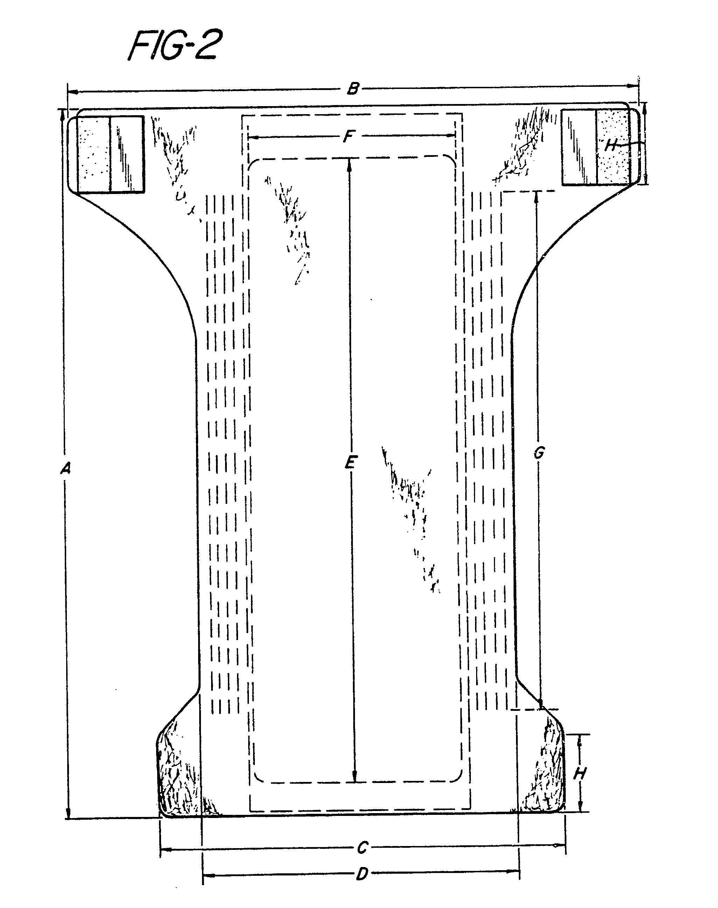

Figure 2 depicts the same diaper but in addition shows the relationship of the measurements of the part of the diaper modified "T" configuration. Assuming that the length of the diaper product A is equal to "x", the width of the back waist portion B is about 0.8x and the width of the front waist portion C is about 0.5x, or slightly more up to 0.55x. The center width D of the product is about 0.45 to 0.51x. The length of the absorbent panel E is about 0.9x, whereas the length of the liquid barrier is substantially equal to x. The width of the absorbent panel F is from about 0.2x to about 0.5x, preferably about 0.25x. The effective elastic length G is about 0.65x. The width of the gathering area of the elastic is at 1.27 cm (1/2 inch) and preferably about 1.90 to 2.54 cm (3/4 to 1 inch). The extension of the diaper at the base of the "T" is equal to the difference in width between the central portion of the diaper and the front waist portion. This difference generally is 0.05x or slightly larger. The length of the top of the "T" H and the extension at the base of the "T" H generally is about 0.1x. The adhesive portion of the closure means extends vertically at least about 0.05x and laterally is about 0.04x or larger. It is important that the vertical adhesion distance be at least about 0.05x and preferably about 0.08x or 0.1x. It is also important that the line from the edge of the top of the "T" toward the stem is a concave line.

The absorbent core of the product is comprised of an absorbent panel and a liquid barrier, the latter of which may be integral with the panel as a layer sprayed thereon or is a liquid barrier sheet slightly larger in area than the absorbent panel. The absorbent panel may be an absorbent batt of lightly compacted cellulosic fibers, such as wood pulp fibers, or a compressed composite product, or the like. The compressed composite product is described in copending European application No. 0 108 637. Briefly, this product consists of an absorbing layer containing superabsorbent and a wicking layer, such as a layer of wood pulp fibers, which layers are compressed to provide a product resulting in an absorbent product which absorbs large quantities of liquid.

The liquid barrier when it is separate from the panel is generally a liquid-impermeable film such as a polyethylene film. The film is of substantially the same shape as the absorbent panel but is slightly larger than the panel in order to be sure that the underside of the absorbent panel is completely covered by the liquid barrier so as to prevent leakage. When the barrier is integral with the panel, such as a latex layer or the like, it extends over at least one surface of the panel.

The backing and facing are of breathable fabrics, such as a spun bonded polypropylene fabric, a polyester nonwoven fabric or a polypropylene melt blown fabric. The backing fabric preferably is substantially liquid-impermeable while the facing fabric is, of course, liquid-permeable.

Conventional elastic means may be used although it is preferred that multiple strands of elastic be placed in the margin of the breathable fabric adjacent the liquid barrier or slightly removed from the edge of the liquid barrier. The elastic extends about 65 percent of the length of the product. However, the elastic is not centered in the length but rather extends closer to the end of the diaper at the back waistband than it does to the front. Specifically, the elastic extends about 55 percent above the center transverse axis and 45 percent below, i.e. toward the front waistband. The effective width of the elastic is from about 0.64 cm (1/4 inch) to about 2.54 cm (1 inch), or more such as 3.81 cm (1-1/2 inches). As heretofore mentioned, the elastic can be a single elastic band, a reticulated elastic band, or multiple strands of elastic which are separated at least 0.16 cm (1/16 inch) one from the other. It has been found that four strands of elastic placed about 0.48 cm (3/16 inch) apart and laminated between the facing and the backing fabric is particularly suitable.

When the absorbent panel is of the conventional type generally made from loosely compacted cellulosic fibers, such as wood pulp fibers, it is particularly desirable to stabilize the absorbent batt. Generally, the wood pulp fibers are quite short, e.g. 0.64 cm (1/4 inch) or less, and are airlaid to form an absorbent batt. The batt may be stabilized by treatment in accordance with U.S. Patent No. 3,017,304, which incorporates in the absorbent batt a densified paper-like layer. This paper-like layer acts as a wick, i.e. liquid which is placed on the layer tends to move rapidly along the plane of the layer. When incorporated in combination with fluffed wood pulp fiber, the resultant product uses the absorbent capacity of the fluffed wood pulp much more efficiently. Other cellulosic fibers that might be used in an absorbent batt are rayon fibers, flax, hemp, jute, ramie, cotton and cotton linters. U.S. Patent Nos. 3,612,055 and 3,938,522 incorporate not only the paper-like layer in a wood pulp fiber batt but also place embossed lines into the batt. An example of the disposable diaper product of the present invention is as follows. This example is not intended to be limiting in any way and extensions and modifications thereof without departure from the scope of the invention will become apparent from the example.

## Example

An absorbent panel is formed having a length of 40.64 cm (16 inches) and a width of 11.43 cm (4.5 inches). The panel is a compressed composite heretofore mentioned. A liquid barrier sheet which is 45.08 cm (17.75 inches) long and 15.87 cm (6.25 inches) wide is placed under the panel. The backing sheet is a spun bonded polypropylene, breathable, substantially liquid-impermeable, nonwoven fabric, which has a length of 45.08 cm (17.75 inches) and a width at the back waistband of 36.83 cm (14.5 inches). The width at the front waistband is 24.13 cm (9.5 inches) and in the center is 21.59 cm (8.5 inches). Four strands of elastic about 0.32 cm (1/8 inch) apart, each of which is 29.21 cm (11.5 inches) long, are placed in the side margin on each side of the absorbent core. The elastic is placed so that 15.87 cm (6.25 inches) of the elastic are in the half of the diaper which forms the back waistband and 13.33 cm (5.25 inches) are in the front half of the diaper. This placement of the elastic forms a soft, but complete, seal about the leg of the infant. A facing also of spun bonded polypropylene, nonwoven fabric is cut to substantially the same shape as the backing and is adhered in the margins to the backing. Integral adhesive closure means are affixed to the diaper ears at the back waistband. The closure means is an adhesive tape repositionable closure. In other words, the diaper may be taken on and off several times using the same tape closure means.

The finished product when placed on an infant does not stretch at the waist because the materials used for the breathable fabric are those which will not yield more than about 10 percent. The improvement in the seal about the leg of the infant is substantial in that the gasketing is very gentle, yet is substantially complete. This is provided by the placement of the multiple strands of elastic giving more elastic gathering toward the back of the diaper than the front as well as the use of the multiple strands of elastic which each provide its own line of gasketing. Furthermore, the modified "T" configuration of the product as shown in Figures 1 and 2 provides a configuration which, whether the absorbent core is thick or thin, fits an infant's torso and legs very well.

The disposable diaper made in accordance with the present invention affords many advantages. Because the product fits the infant, the clothing placed over the diaper fits much better. The breathability of the backing and facing fabrics reduces incidents of skin irritation. Also the soft gathering provided by elastic effective over a width of at least 1.27 cm (1/2 inch), reduces irritation to the skin. The all fabric backing and facing prevents perspiration on the arm of one carring the infant. Other advantages of the product include reduced leakage and improved comfort.

From the foregoing it will be observed that numerous variations and modifications may be effected without departing from the scope of the invention as defined in the claims.

## Claims

1. A disposable infant diaper comprising:
   (a) an absorbent panel (16);
   (b) a first breathable fabric (12) on which the absorbent panel (16) is located, the first breathable fabric extending beyond the longitudinal edges of the absorbent panel (16) to form margins;
   (c) a second breathable fabric (18) covering said absorbent panel (16) and extending beyond said panel (16) at least sufficiently to be adhered to said first breathable fabric in the margins;
   (d) elastic gathering means (13) secured between said first (12) and second (18) breathable fabrics in said margins along the longitudinal length of said absorbent panel (16) in at least the central portion thereof;

and
(e) closure means (17, 19) attached on the inside of the diaper at one end and on either side of said diaper,

characterised in that:
(f) the diaper has a modified "T" configuration;
(g) the absorbent panel (16) is substantially rectangular;
(h) there is a liquid barrier (14) between said absorbent panel (16) and said first breathable fabric (12), said barrier (14) being substantially the same shape as said panel (16) but extending slightly beyond each edge of said panel (16);
(i) the liquid barrier (14) and the absorbent panel (16) together form an absorbent core, which core forms the stem of the "T" and extends substantially the entire length of the "T";
(j) the first breathable fabric (12) forms the top of the "T";
(k) the closure means (17, 19) are attached at respective ends of the top of the "T";
(l) extended portions of at least one of said breathable fabrics (12, 18) are provided at the base of the "T" along a small portion of the stem of the "T" on each side in substantially equal portions; and
(m) said breathable fabrics (12, 18) are substantially non-extendible in a cross-wise direction.

2. The disposable diaper of claim 1, wherein:
the length of the "T" configuration is x;
the width across the top of the "T" is about 0.8x;
the width of the base of the stem of the "T" is from 0.5x to 0.55x;
the width of the stem of the "T" at the transverse axis at the center of the length of the product is from 0.45x to 0.50x;
the length of the panel (16) is about 0.9x;
the width of the panel (16) is about 0.25x;
the length of the elastic gathering means (13) is about 0.65x;
said elastic gathering means (13) is distributed with about 55 percent of its length above the transverse axis and about 45 percent of its length below the transverse axis;
said elastic means (13) is at least about 1.27 cm (0.5 inch) in width;
said closure means (17, 19) longitudinal length is at least about 0.08x; and
the terminating ends of the top of the "T" are about 0.1x in length.

3. The disposable diaper of claim 1 or claim 2, wherein said absorbent panel (16) comprises a batt of loosely compacted cellulosic fibers.

4. The disposable diaper of claim 3, wherein said cellulosic fibers are wood pulp fibers.

5. The disposable diaper of claim 1 or claim 2, wherein said absorbent panel (16) is comprised of a compressed composite containing superabsorbent.

6. The disposable diaper of any one of claims 1 to 5, wherein said first and second breathable fabrics (12, 18) comprise a spun bonded polypropylene nonwoven fabric.

7. The disposable diaper of any one of claims 1 to 6, wherein said elastic gathering means (13) comprises multiple strands of elastic.

8. The disposable diaper of claim 7, wherein said strands of elastic (13) are substantially parallel to each other and are at least about 0.16 cm (1/16 inch) apart.

9. The disposable diaper of any one of claims 1 to 8, wherein said liquid barrier (14) comprises polyethylene film.

10. The disposable diaper of any one of claims 1 to 9, wherein said closure means are adhesive closure means (17, 19).

11. The disposable diaper of claim 10, wherein said adhesive closure means (17, 19) are masses of adhesive deposited on the fabric at the ends of the top of the "T".

**Revendications**

1. Une couche jetable pour bébé comprenant :
   (a) un panneau absorbant (16) ;
   (b) un premier tissu aéré (12) sur lequel est situé le panneau absorbant (16), le premier tissu aéré s'étendant au delà des bords longitudinaux du panneau absorbant (16) pour former des marges ;
   (c) un second tissu aéré (18) recouvrant ledit panneau absorbant (16) et s'étendant au delà dudit panneau (16) d'une façon au moins suffisante pour être collé audit premier tissu aéré dans les marges ;
   (d) des moyens de fronçure élastiques (13) fixés entre lesdits premier (12) et second (18) tissus aérés dans lesdites marges le long de la longueur longitudinale dudit panneau absorbant (16), au moins dans leurs parties centrales ; et

(e) des moyens de fermeture (17,19) fixés sur l'intérieur de la couche à une extrémité et sur les deux côtés de ladite couche, caractérisée en ce que :

(f) la couche a une configuration en "T" modifié ;

(g) le panneau absorbant (16) est substantiellement rectangulaire ;

(h) il y a une barrière aux liquides (14) entre ledit panneau absorbant (16) et ledit premier tissu aéré (12), ladite barrière (14) ayant substantiellement la même forme que ledit panneau (16) mais s'étendant légèrement au delà de chaque bord dudit panneau (16) ;

(i) la barrière aux liquides (14) et le panneau absorbant (16) formant ensemble une partie centrale absorbante, ladite partie centrale absorbante formant la tige du "T" et s'étendant substantiellement sur toute la longueur du "T" ;

(j) le premier tissu aéré (12) forme le haut du "T" ;

(k) les moyens de fermeture (17, 19) sont fixés aux extrémités respectives du haut du "T" ;

(l) des parties allongées d'au moins l'un desdits tissus aérés (12, 18) sont fournies à la base du "T" le long d'une petite partie de la tige du "T" sur chaque côté formant des parties substantiellement égales ; et

(m) lesdits tissus aérés (12, 18) sont substantiellement non extensibles dans le sens transversal.

2. La couche jetable pour bébé selon la revendication 1, dans laquelle :

la longueur de la configuration en "T" est x ;

la largeur en travers du haut du "T" est d'environ 0,8 x ;

la largeur de la base de la tige du "T" va de 0,5 x à 0,55 x ;

la largeur de la tige du "T" au niveau de l'axe transversal au centre de la longueur du produit va de 0,45 x à 0,50 x ;

la longueur du panneau (16) est d'environ 0,9 x ;

la largeur du panneau (16) est d'environ 0,25 x ;

la longueur des moyens de fronçure élastiques (13) est d'environ 0,65 x ;

lesdits moyens de fronçure élastiques (13) sont répartis de sorte qu'environ 55 pour cent de leur longueur est située au dessus de l'axe transversal et environ 45 pour cent de leur longueur est située au dessous de l'axe transversal ;

lesdits moyens élastiques (13) ont une largeur d'au moins environ 1,27 cm (0,5 pouce) ;

la longueur longitudinale desdits moyens de fermeture (17, 19) est d'au moins environ 0,08 x ; et

les extrémités de fin du haut du "T" ont une longueur d'environ 0,1 x.

3. La couche jetable pour bébé selon la revendication 1 ou la revendication 2, dans laquelle le panneau absorbant (16) comprend un molleton de fibres cellulosiques lâchement compactées.

4. La couche jetable pour bébé selon la revendication 3, dans laquelle lesdites fibres cellulosiques sont des fibres de pulpe de bois.

5. La couche jetable pour bébé selon la revendication 1 ou la revendication 2, dans laquelle ledit panneau absorbant (16) est composé d'un superabsorbant contenant un composite comprimé.

6. La couche jetable pour bébé selon l'une des revendications 1 à 5, dans laquelle lesdits premier et second tissus aérés (12, 18) comprennent un tissu non tissé de polypropylène lié par câblage.

7. La couche jetable pour bébé selon l'une des revendications 1 à 6, dans laquelle lesdits moyens de fronçure élastiques (13) comprennent des brins multiples d'élastique.

8. La couche jetable pour bébé selon la revendication 7, dans laquelle lesdits brins d'élastique (13) sont substantiellement parallèles les uns aux autres et sont espacés d'au moins environ 0,16 cm (1/16$^{ème}$ de pouce).

9. La couche jetable pour bébé selon l'une des revendications 1 à 8, dans laquelle ladite barrière aux liquides (14) comprend un film de polyéthylène.

10. La couche jetable pour bébé selon l'une des revendications 1 à 9, dans laquelle lesdits moyens de fermeture sont des moyens de fermeture adhésifs (17, 19).

11. La couche jetable pour bébé selon la revendication 10, dans laquelle lesdits moyens de fermeture adhésifs (17, 19) sont des masses d'adhésif déposées sur le tissu aux extrémités du haut du "T".

## Ansprüche

1. Wegwerfbare Babywindel,
   bestehend aus:
   (a) einer Absorptionsschicht (16);
   (b) einem luftdurchlässigen Stoff (12), auf dem die Absorptionsschicht (16) angeordnet ist, wobei der erste luftdurchlässige Stoff sich über die Längsränder der Absorptionsschicht (16) zur Bildung von Rändern erstreckt;
   (c) einem zweiten luftdurchlässigen Stoff (18), der die Absorptionsschicht (16) abdeckt und sich über diese Schicht (16) zumindest soweit erstreckt, daß der zweite Stoff an den Rändern des ersten luftdurchlässigen Stoffes festhaftet;
   (d) einem elastischen Zugmittel (13), das zwischen den ersten (12) und zweiten (18) luftdurchlässigen Stoffen an deren Rändern in mindestens dem mittleren Teil derselben in Längsrichtung der Absorptionsschicht (16) befestigt ist; und
   (e) Verschlußmitteln (17, 19), die an der Innenseite der Windel an einem Ende und an beiden Seiten der Windel angebracht sind,
   dadurch gekennzeichnet, daß
   (f) die Windel eine modifizierte "T"-Form hat;
   (g) die Absorptionsschicht (16) im wesentlichen rechtwinklig ist;
   (h) eine Flüssigkeitssperrschicht (14) zwischen der Absorptionsschicht (16) und dem ersten luftdurchlässigenStoff (12) vorgesehen ist, wobei die Sperrschicht (14) im wesentlichen die gleiche Form wie die Schicht (16) hat, aber sich etwas über jeden Rand der Schicht (16) hinaus erstreckt;
   (i) die Flüssigkeitssperrschicht (14) und die Absorptionsschicht (16) zusammen einen Absorptionskern bilden, wobei der Kern den Schaft des "T" bildet und sich im wesentlichen über die gesamte Länge des "T" erstreckt;
   (j) das erste luftdurchlässige Stoff (12) den Kopf des "T" bildet;
   (k) die Schließmittel (17, 19) an den entsprechenden Enden des Kopfes des "T" angebracht sind;
   (l) breitere Teile mindestens eines der luftdurchlässigen Stoffe (12, 18) an der Basis des "T" längs eines kleinen Teils des Schaftes des "T" an jeder Seite in im wesentlichen gleichen Teilen vorgesehen sind; und
   (m) die luftdurchlässigen Stoffe (12, 18) in Querrichtung im wesentlichen nicht dehnbar sind.

2. Wegwerfwindel nach Anspruch 1, bei der:
   die Länge der "T"-Form x ist;
   die Breite des Kopfes des "T" etwa 0,8 x ist;
   die Breite der Basis des Schaftes des "T" von 0,5 x bis 0,55 x beträgt;
   die Breite des Schaftes des "T" in der Längsmitte des Erzeugnisses von 0,45 x bis 0,50 x beträgt;
   die Länge der Schicht (16) etwa 0,9 x ist;
   die Breite der Schicht (16) etwa 0,25 x ist;
   55 % der Länge der elastischen Zugmittel (13) auf den oberhalb der Querachse liegenden Teil und etwa 45 % ihrer Länge auf den unterhalb der Querachse liegenden Teil verteilt sind;
   die elastischen Mittel (13) mindestens etwa 1,27 cm (0,5 Zoll) breit sind,
   das die Längserstreckung der Verschlußmittel (17, 19) mindestens etwa 0,08 x beträgt; und
   die Abschlußenden der Oberseite des "T" etwa 0,1 x lang sind.

3. Wegwerfwindel nach Anspruch 1 oder Anspruch 2, bei der die Absorptionsschicht (16) eine Bahn von lose kompaktierten, cellulosehaltigen Fasern umfaßt.

4. Wegwerfwindel nach Anspruch 3, bei der die genannten cellulosehaltigen Fasern Holzpulpefasern sind.

5. Wegwerfwindel nach Anspruch 1 oder Anspruch 2, bei der die Absorptionsschicht (16) aus einem zusammengepreßten Verbundstoff besteht, der ein superabsorbierendes Material enthält.

6. Wegwerfwindel nach einem der Ansprüche 1 bis 5, bei der die ersten und zweiten luftdurchlässigen Stoffe (12, 18) einen Nonwoven-Stoff aus durch Spinnen gebundenem Polypropylen umfassen.

7. Wegwerfwindel nach einem der Ansprüche 1 bis 6, bei der das elastische Zugmittel (13) aus einer Mehrzahl von elastischen Strängen besteht.

8. Wegwerfwindel nach Anspruch 7, bei der die elastischen Stränge (13) im wesentlichen parallel zueinander verlaufen und einen Abstand von mindestens etwa 0,16 cm (1/16 Zoll) aufweisen.

9. Wegwerfwindel nach einem der Ansprüche 1 bis 8, bei der die Flüssigkeitssperrschicht (14) aus einem Polyethylenfilm besteht.

**10.** Wegwerfwindel nach einem der Ansprüche 1 bis 9 bei der das Verschlußmittel ein Klebverschlußmittel (17, 19) ist.

**11.** Wegwerfwindel nach Anspruch 10, bei der das Klebverschlußmittel (17, 19) Klebstoffmassen sind, die auf dem Stoff an den Enden der Oberseite des "T" aufgebracht sind.

FIG-1

## FIG-2